Europäisches Patentamt

European Patent Office

Office européen des brevets

(11) Publication number: **0 169 549 B1**

# EUROPEAN PATENT SPECIFICATION

(45) Date of publication of patent specification: **02.09.92**

(51) Int. Cl.⁵: **G01N 33/573**, G01N 33/543, G01N 33/577

(21) Application number: **85109227.0**

(22) Date of filing: **23.07.85**

The file contains technical information submitted after the application was filed and not included in this specification

(54) **Immunological determination of human plasmin/alpha2-plasmin inhibitor.**

(30) Priority: **26.07.84 JP 153993/84**
**20.03.85 JP 54208/85**

(43) Date of publication of application:
**29.01.86 Bulletin 86/05**

(45) Publication of the grant of the patent:
**02.09.92 Bulletin 92/36**

(84) Designated Contracting States:
**BE CH DE FR GB LI SE**

(56) References cited:
**EP-A- 0 038 935**
**BE-A- 896 543**
**DE-A- 2 711 164**

**J. CLIN. INVEST., vol.68, July 1981, American Society for Clinical Investigation Inc. (US); P.C.HARPEL, pp.46-55**

**BIOSCIENCE REPORTS, vol.4, January 1984, The Biochemical Society (GB); P.HERION et al., pp.39-48**

(73) Proprietor: **TEIJIN LIMITED**
**11 Minamihonmachi 1-chome Higashi-ku**
**Osaka-shi Osaka 541(JP)**

(72) Inventor: **Sumi, Yoshihiko**
**18-4, Tamadaira 3-chome**
**Hino-shi Tokyo(JP)**
Inventor: **Koike, Yukiya**
**5-18, Tamadaira 3-chome**
**Hino-shi Tokyo(JP)**
Inventor: **Ichikawa, Yataro**
**11-7, Kotesashi-cho 2-chome**
**Tokorozawa-shi Saitama-ken(JP)**
Inventor: **Yoshida, Nobuhiko, Jichiidai-Shokuin-Jutaku 31-8**
**3311-158, Oaza Yakushiji, Minamikawachi-cho**
**Kawachi-gun, Tochigi-ken(JP)**
Inventor: **Aoki, Nobuo**
**20-2-304, Hongo 4-chome Bunkyo-ku**
**Tokyo(JP)**

(74) Representative: **Kraus, Walter, Dr. et al**
**Patentanwälte Kraus, Weisert & Partner**
**Thomas-Wimmer-Ring 15**
**W-8000 München 22(DE)**

## Description

This invention relates to the immunological determination of a human plasmin/$\alpha_2$-plasmin inhibitor complex (or a human plasmin/$\alpha_2$-antiplasmin complex) in an assay sample. More specifically, it relates to a method of quantitatively determining a human plasmin/$\alpha_2$-plasmin inhibitor complex in an assay sample such as a serum sample by the sandwich technique using a monoclonal antibody specific to both a human $\alpha_2$-plasmin inhibitor and a human plasmin/$\alpha_2$-plasmin inhibitor and an antibody specific to human plasmin, and a reagent system used therefor.

It is known that the human $\alpha_2$-plasmin inhibitor (to be abbreviated hereinafter as "human $\alpha_2$-PI") is a single-chain glycoprotein having a carbohydrate content of 11.7% and a molecular weight of about 67,000 which was first isolated in pure form from human plasma by Aoki and Moroi and acts as a strong inhibitor capable of instantaneously inhibiting the esterase activity of plasmin, a fibrinolytic enzyme [see Moroi & Aoki: The Journal of Biological Chemistry, 251, 5956-5965 (1976)].

Human $\alpha_2$-PI has three functions. Firstly, it has a site of inhibiting the fibrinolytic activity of plasmin (in the present specification, this site is referred to as the "reactive site") [see B. Wiman & D. Collen: The Journal of Biological Chemistry, 254, 9291-9297 (1979)]. Secondly, it has a site combining with plasmin at the carboxyl group terminal [B. Wiman & D. Collen: European Journal of Biochemistry, 84, 573-578 (1978)]. Thirdly, it has a site combining with fibrin at the amino group terminal [Y. Sakata et al.: Thrombosis Research, 16, 279-282 (1979)].

Human $\alpha_2$-PI almost instantaneously inhibits the activity of plasmin, and combines with plasmin at a ratio of 1:1 to form a complex [see B. Wiman & D. Collen: The Journal of Biological Chemistry, 254, 9291-9297 (1979); D. Collen: Thrombosis and Haemostasis, 43, 77-89 (1980)].

In the blood of patients in which plasminogen is in the activated state, such as patients with disseminated intravascular coagulation (DIC) or patients under thrombolytic therapy with urokinase, a part of the plasmin formed by the activation of plasminogen reacts with $\alpha_2$-PI to form a complex. The determination of a plasmin/$\alpha_2$-PI complex in the plasma is considered to be effective for monitoring the thrombolytic therapy or for diagnosing DIC [see, for example, N. A. Booth & B. Bennett: British Journal of Haematology, 50, 537-541 (1982)].

If, therefore, the amount of a plasmin/$\alpha_2$-PI complex in the blood can be accurately and easily measured, it will be very useful for preventing and diagnosing various diseases.

Known techniques of measuring a plasmin/$\alpha_2$-PI complex include a method involving the use of second dimensional crossed immunoelectrophoresis [see, for example, N. A. Booth & Bennett, British Journal of Haematology, 50, 537-541 (1982)], and a so-called sandwich method in which a polyclonal antibody obtained from an antiserum is fixed and an enzyme-antibody method is applied [see, for example, P. C. Harpel, Journal of Clinical Investigation, 68, 46-55 (1981)].

However, the former method has the defect of low sensitivity and quantitativeness. The latter method has a relatively high sensitivity, but has the defect that an antiserum having a certain activity on human $\alpha_2$-PI is difficult to obtain stably.

The present inventors considered a monoclonal antibody as a candidate for an antiserum having a fixed activity on $\alpha_2$-PI, and worked extensively on the creation of monoclonal antibodies specific to $\alpha_2$-PI, and a method of stably producing such monoclonal antibodies. Consequently, the inventors have found some monoclonal antibodies which specifically combine with $\alpha_2$-PI, and succeeded in creating hybridoma cells which stably produce such monoclonal antibodies. It has been found that the use of monoclonal antibodies so produced enables a human plasmin/$\alpha_2$-PI complex in an assay sample to be determined with a very high sensitivity and good reproducibility by the sandwich method.

Thus, according to this invention, there is provided a method for immunologically determining a human plasmin/$\alpha_2$-plasmin inhibitor complex in an assay sample by using a primary antibody fixed to an insoluble solid carrier and a labelled secondary antibody, wherein the secondary antibodies is a monoclonal antibody or its fragment specific to both a human $\alpha_2$-plasmin inhibitor and a human plasmin/$\alpha_2$-plasmin inhibitor complex, and the primary antibody is a polyclonal antibody specific to human plasmin.

Generally, a method of determining the presence or absence of an antigen or measuring its amount by using antibodies which combine with two different sites of the antigen is called the "sandwich method", and is described, for example, in Wide's Radioimmunoassay Methods, 199-206 (1970).

The immunological assay method of this ivnention uses two antibodies (primary and secondary antibodies) the secondary of which is a monoclonal antibody that specifically recognizes, and combine with, both human $\alpha_2$-PI and a human plasmin/$\alpha_2$-PI complex, and the primary of which is an antibody specific to human plasmin.

In the method of this invention, a fragment of the monoclonal antibody at least containing a Fab region having an antigen binding site (variable region) may also be used. Accordingly, it should be understood that unless otherwise stated, the term "antibody" used in the present specification also denotes its fragment at least containing the Fab region.

Thus, the method of this invention enables a human plasmin/$\alpha_2$-PI complex in an assay solution, for example in a plasma sample, to be always determined highly accurately without changes in the quality of the reagent used. Since the amount of human plasmin/$\alpha_2$-PI complex is directly measured, the method is not at all affected by foreign materials and can determine the human plasmin/$\alpha_2$-PI complex accurately within short periods of time. Accordingly, the present invention provides a new method of determining human $\alpha_2$-PI accurately and rapidly.

In the method of this invention, one (primary antibody) of the two antibodies is fixed to an insoluble solid carrier, and the other (secondary antibody) is used in the labelled state. The monoclonal antibody is used as the labelled secondary antibody.

The primary antibody may be fixed to the insoluble solid carrier by methods known per se. For example, a solution of the primary antibody and the insoluble solid carrier are contacted and left to stand, whereby the antibody is physically adsorbed on the carrier. It is also possible to combine the functional groups of the antibody, such as a carboxyl, amino or hydroxyl group, chemically with the insoluble solid carrier. Preferably, the surface of the carrier to which the primary antibody has been fixed is coated with a suitable substance such as bovine serum albumin to avoid non-specific combination with the secondary antibody or the assay sample.

Examples of the insoluble solid carrier used to fix the primary antibody include polymeric materials such as polystyrene, polyethylene, polypropylene, polyesters, polyacrylonitrile, fluorine-containing resins, nitrocellulose, crosslinked dextran, polysaccharides and agarose, inorganic materials such as glass and metal, and combinations of these. The solid carrier may be in various shapes, for example, in the shape of a tray, sphere, fiber, particle, bead, disc, rod, receptacle, cell or test tube. Specific examples of the insoluble solid carrier are plastic receptacles, plastic beads, glass beads and metal particles.

The secondary antibody is labelled with radioisotopes, enzymes or luminescent substances. Examples of the radioisotopes are $^{125}$I, $^{131}$I, $^{14}$C and $^3$H. Examples of the enzymes are alkaline phosphatase, peroxidase, and beta-D-galactosidase. Examples of the luminescent substances are fluorescein isothiocyanate and tetramethyl rhodamine isothiocyanate. These are merely illustrative, and other labelling substances used in immunological assay may also be used. Combination of the labelling substances with the secondary antibody may be carried out by methods known per se, for example by the methods described in G. S. David: Biochem. Biophys. Res. Commun., 48, 464-471 (1972), M. Imagawa et al., Anal. Lett., 16, 1509-1523 (1983) and M. Nishioka et al., Cancer Res., 32, 162-166 (1972).

The fixed primary antibody and the labelled secondary antibody are then brought into contact with an assay sample for determination of the human plasmin/$\alpha_2$-PI complex by a two-step method comprising contacting the sample first with the fixed primary antibody and then with the labelled secondary antibody, or by a one-step method comprising contacting the sample and the secondary antibody simultaneously with the primary antibody. The one-step method, however, is advantageous over the two-step method because it permits a simpler and more rapid determination of the human plasmin/$\alpha_2$-PI complex.

In the two-step method, the fixed primary antibody and the sample are contacted and reacted at a given temperature for a given period of time. During this time, the fixed primary antibody combines with the human plasmin/$\alpha_2$-PI complex in the sample. After washing with a suitable washing liquor, the reaction product is contacted and reacted with a solution (e.g., an aqueous solution) of the labelled secondary antibody at a given temperature for a given period of time. The reaction product is washed with a suitable washing liquid, and the amount of the labelling substance present on the insoluble solid carrier is measured. The amount of the human plasmin/$\alpha_2$-PI complex in the sample can be determined by comparing the amount of the labelling substance with a calibration curve drawn by using an assay sample containing the human plasmin/$\alpha_2$-PI complex in a known concentration.

In the one-step method, the fixed primary antibody is contacted and reacted with the assay sample and the labelled secondary antibody simultaneously, preferably with a mixture of the sample and the labelled secondary antibody at a given temperature for a given period of time. The product is then washed with a suitable washing liquid, and the amount of the labelling substance which is present on the insoluble solid carrier is measured as described above. As a result, the amount of the human plasmin/$\alpha_2$-PI complex in the sample can be determined.

According to the methods described above, the amount of the human plasmin/$\alpha_2$-PI complex in the assay sample can be measured easily with good reproducibility and high accuracy. Human plasma, human serum and a supernatant from a cell culture are examples of the sample which can be assayed by the above methods.

For the practice of the above method, the present invention provides a reagent system comprising the primary, polyclonal antibody fixed to the insoluble solid carrier and the labelled secondary, monoclonal antibody. A kit may be formed from this reagent system and various auxiliary agents in order to use the reagent system efficiently and easily. Examples of the auxiliary agents include dissolving agents for dissolving the solid secondary antibody, washing agents for washing the insoluble carrier, substrates for measuring the enzyme activity of enzymes which may be used as labelling substances for the secondary antibody, and reaction stoppers therefor, which are normally used in reagent kits for immunological assay.

The monoclonal antibody specific to both a human $\alpha_2$-PI and a human plasmin/$\alpha_2$-PI complex can be obtained by establishing a hybridoma cell line capable of producing the above antibody, and cultivating the hybridoma.

The hybridoma capable of producing the monoclonal antibody can be produced by a technique known as the Köhler and Milstein method [Köhler and Milstein, Nature, 256, 495-497 (1975)]. Specifically, a mammal such as a mouse is immunized with human $\alpha_2$-PI, and antibody-producing cells, for example, spleen cells, of this animal are fused with myeloma cells. The fused cells are screened by cloning for fused cells capable of producing the monoclonal antibody. For example, the fused cells produced are systematically screened for an antibody which reacts with human $\alpha_2$-PI fixed to microtiter plates. In this way, hybridoma cells which synthesize and secrete an antibody to $\alpha_2$-PI are selected. The resulting hybridoma cells are cultivated in a medium containing or not containing serum. Antibodies specific to human $\alpha_2$-PI secreted in a supernatant from the culture fluid are systematically examined and selected against an antibody which react with the human plasmin/$\alpha_2$-PI complex fixed to the microtiter plates. As a result, a monoclonal antibody specific to both the human $\alpha_2$-PI and the human plasmin/$\alpha_2$-PI complex can be isolated.

The monoclonal antibody can be obtained from the product yielded by this hybridoma. The resulting monoclonal antibody acts monospecifically on that site of the human plasmin/$\alpha_2$-PI complex which is not blocked with plasmin.

The monoclonal antibody and a process for its production will now be described in detail.

(A) Isolation and purification of antigen

Human $\alpha_2$-PI used as an antigen is isolated in pure form from a human plasma sample by the aforesaid method of Aoki and Moroi.

(B) Immunization of mammals with human $\alpha_2$-PI

There is no particular restriction on the animals to be immmunized, and various mammals such as mice, rats, guinea pigs, rabbits, sheep, goats, dogs and cats may be used. For the ease of handling, male Balb/c mice are generally used. Mice of other strains may also be used. The immunization should be planned, and the concentration of human $\alpha_2$-PI to be used in immunization should be selected, so that sufficient amounts of antigenically stimulated lymphocytes can be formed. For example, a mouse is intraperitoneally immunized several times with a small amount of human $\alpha_2$-PI at certain intervals, and the antigen is further administered intravenously several times to increase the titer of the antibody. Several days after the final immunization, antibody-producing cells, for example, lymphocytes, preferably spleen cells, are taken out from the immunized animals. The following description is given with regard to the use of spleen cells as the antibody-producing cells, but it should be understood that other antibody-producing cells isolated from immunized animals can equally be used for cell fusion.

(C) Cell fusion

The spleen is aseptically taken out from the immunized animal, and a spleen cell suspension is prepared from it. The spleen cells are then fused with myeloma cells taken from a suitable cell line in a fusion medium in the presence of a suitable fusion promoter. The myeloma cells used for fusion may be obtained from any mammals, but generally, those originated from the same kind of animal as the immunized animal are preferred. The preferred mixing ratio between the spleen cells and the myeloma cells is generally in the range of from about 20:1 to about 2:1, preferably from 10:1 to 2:1. Usually, the use of 0.5 to 1.5 ml of the fusion medium per about $10^8$ spleen cells is suitable. Suitable fusion media are, for example, physiological saline, buffered saline, a serum-free medium each of which contains the fusion promoter in a concentration of 30 to 70%.

Many myeloma cells suitable for cell fusion are known. In Examples to be given hereinafter, P3-X63-

Ag8-UI cells (to be abbreviated as P3-UI) [see D. E. Yelton et al.: Current Topics in Microbiology and Immunology, 81, I (1978)]. They are an 8-azaguanine resistance cell line. They lack hypoxanthine-guanine phosphoribosyl transferase, and therefore do not survive in HAT medium (containing hypoxanthine, aminopterin and thymidine). Furthermore, since this cell line is of a non-secreting type which does not secrete an antibody itself, it is suitable for the production of the hybridoma. Other myeloma cells may also be used. Examples include P3-NSI-I-Ag4-I, NSI-Ag4/I, P3-X63-Ag8, (MPCH-45, 6. TGI.7), SP2/0-AgI4, FO, X-63-Ag8-6.5.3, 210.RCY3.AgI.2.3, SI94/5XXO.BU.I, SKO-007, and GMI5006TG-AI2.

Polyethylene glycol having an average molecular weight of 1,000 to 4,000, for example, may be advantageously used as the fusion promoter. There can also be used other fusion promoters known in the art, such as Sendai virus. In the following Examples, polyethylene glycol having an average molecular weight of 1,540 was used.

(D) Detection of the fused cells

A mixture of the fused cells, non-fused spleen cells and non-fused myeloma cells is diluted in a separate receptacle (such as a microtiter plate) with a selective medium in which the non-fused myeloma cells cannot survive, and cultivated for a sufficient period of time to allow the non-fused cells to die (about 1 week). The culture medium may be one which is resistant to a drug such as 8-azaguanine and in which the non-fused myeloma cells cannot survive, for example the aforesaid HAT medium. In the selective medium, the non-fused myeloma cells die away. Since the non-fused spleen cells are non-tumoral, they die after a certain period of time (about 1 week). On the other hand, the fused cells can survive in the selective medium because they have both the tumor-bearing nature of the parent myeloma cells and the nature of the parent spleen cells.

(E) Determination of an antibody to human $\alpha_2$-PI in each receptacle

After the hybridoma cells are detected as stated above, the supernatant of the culture fluid is collected, and screened for an antibody to human $\alpha_2$-PI by enzyme linked immunosorbent assay (see, for example, A. H. W. M. Schuurs and B. K. van Weemen: Clin. Chim. Acta, 81, I-40 (1977)].

(F) Cloning of the hybridoma capable of producing an antibody specific to human $\alpha_2$-PI

The hybridoma capable of producing an antibody specific to human $\alpha_2$-PI can be cloned by a suitable method such as a limiting dilution method in two different ways. In one way, the hybridoma is cultivated in a suitable medium for a given period of time, and the monoclonal antibody produced by the hybridoma can be obtained from the supernatant of the culture fluid. In the other, the hybridoma can be intraperitoneally injected into a syngenic mouse. After a certain period of time, the monoclonal antibody produced by the hybridoma can be obtained from the blood and ascites of the host animal.

(G) Screening of the desired antibody

Purified monoclonal antibodies to human $\alpha_2$-PI were screened for an antibody specific to a human plasmin/$a_2$-PI complex by enzyme linked immunosorbent assay [see, for example, A. H. W. M. Schuurs and B. K. van Weemen: Clin. Chim. Acta, 81, I-40 (1977)].

Among the monoclonal antibodies so produced, a monoclonal antibody specific to a fragment of human $\alpha_2$-PI molecule which has a length extending about 12,000 in molecular weight from the carboxylic terminal of the molecule is preferred from the standpoint of the detecting sensitivity of the human plasmin/$\alpha_2$-PI complex and reproducibility. Especially preferred is a monoclonal antibody having the function of specifically blocking the reactive site of human $\alpha_2$-PI, i.e. that site of human $\alpha_2$-PI which inhibits the fibrinolytic activity of plasmin, and suppressing the inherent action of human $\alpha_2$-PI to inhibit the fibrinolytic activity of plasmin.

In the present specification, the expression "blocks the reactive site" means the addition or combination of the monoclonal antibody to or with the reactive site of human $\alpha_2$-PI in such a way that the monoclonal antibody recognizes the reactive site itself or any of the epitopes of human $\alpha_2$-PI thereby to cause the reactive site to lose activity.

Investigations of the present inventors have led to the belief that the monoclonal antibody having the function of specifically blocking the reactive site of human $\alpha_2$-PI recognizes, and combines with, a site about 8000 in molecular weight away from the carboxylic terminal of the human $\alpha_2$-PI molecule.

Such a monoclonal antibody can be created by performing the following step (E') before the above step (F).

(E') The supernatant of the culture fluid obtained by cultivating the hybridoma producing an antibody to human $\alpha_2$-PI is concentrated and incubated with the human $\alpha_2$-PI for a fixed period of time. Plasmin is added to the mixture, and the mixture is placed on a fibrin plate. The area of the fibrin dissolved is measured. In this way, a hybridoma capable of producing an antibody having activity on human $\alpha_2$-PI is selected.

It is believed that the monoclonal antibody produced by the above method, as other antibodies do, has in its variable region an antigen binding site capable of performing the aforesaid function.

The resulting monoclonal antibody is cleaved by the Porter's method [see R. R. Porter, Biochemical Journal, 73, 119-126 (1959)] using papain, a proteolytic enzyme, and a "Fab"-containing fragment can be isolated.

The Fab-containing fragment of the monoclonal antibody has been examined on microtiter plates for the ability to combine with human $\alpha_2$-PI and the human plasmin/$\alpha_2$-PI complex. It has consequently been ascertained that only the Fab-containing fragment of the monoclonal antibody has the same antibody function as the monoclonal antibody.

In the present invention, therefore, the Fab-containing fragment can be used instead of the monoclonal antibody itself. Such a fragment includes, for example, not only a papain-cleaved fragment but also other fragments containing the Fab region obtained after cleavage with trypsin, plasmin, etc.

In the immunological assay in accordance with this invention, the above monoclonal antibody or its Fab-containing fragment is used either as the primary or secondary antibody. Specifically, it may be used as the primary antibody by bonding it to an insoluble solid carrier, or as the secondary antibody by labelling it. Generally, it is economic and convenient to use it as the labelled secondary antibody.

The antibody specific to human plasmin used in combination with the monoclonal antibody or its fragment may be monoclonal or polyclonal if it has the function of recognizing, and combining with, the human plasmin. From the standpoint of the ease of availability, the polyclonal antibody is generally used.

Such an antibody specific to human plasmin is known per se, and can be produced by methods known per se [see, for example, A. Johnstone & R. Thorpe: Immunochemistry in Practice, Blackwell Scientific Publications, 27-31]. For example, an animal such as rabbit, is immunized with human plasminogen to raise its antibody titer fully, and the whole blood or a partial blood is extracted. An antiserum is separated from the extracted blood sample, fractionated with ammonium sulfate, and fully dialyzed. By DEAE-cellulose ion-exchange chromatography, only IgG flows out without adsorption. This effluent fraction is concentrated and dialyzed against a suitable buffer such as phosphate-buffered saline to obtain an anti-human plasminogen sample having a suitable concentration for use [see, for example, H. A. Sober, F. J. Gutter, M. M. Wyckoff and E. A. Peterson: J. Amer. Chem. Soc. 78, 751 (L956)].

The primary reason for using human plasminogen instead of human plasmin for immunization is that plasmin is a proteolytic enzyme and is susceptible to deactivation and is less stable than plasminogen, its precursor. When The Arg-Val bond on the COOH terminal side of plasminogen is cleaved and a peptide composed of 77 to 78 residues on the $NH_2$ terminal side is liberated, plasminogen becomes double-stranded plasmin bonded by an S-S linkage. Accordingly, an anti-human plasminogen antibody obtained by immunizing human plasminogen can combine with both human plasminogen and plasmin.

According to the method and the reagent system of this invention, the amount of a human plasmin/$\alpha_2$-PI complex in an assay sample containing the complex, for example, a human plasma sample, can be determined accurately and easily with good reproducibility and high sensitivity.

The present inventors previously found that the monoclonal antibody or its fragment having the function of specifically blocking the reactive site of human $\alpha_2$-PI which inhibits the fibrinolytic activity of plasmin, and of suppressing the fibrinolytic activity inhibiting function of $\alpha_2$-PI is useful for the immunological determination of not only the human plasmin/$\alpha_2$-PI complex but also human $\alpha_2$-PI itself in an assay sample by the sandwich method. Consequently, the inventors disclosed and claimed the method and reagent system for immunologically determining a human $\alpha_2$-PI in an assay sample using the aforesaid monoclonal antibody or its fragment in the copending application EP-A-0159 025. The reagent system disclosed in the copending application comprises a primary antibody fixed to an insoluble solid carrier and a labelled secondary antibody, wherein the primary and secondary antibodies are anti-human $\alpha_2$-PI antibodies or their fragments which specifically recognize and combine with different epitopes of the human $\alpha_2$-PI, and one of them is the monoclonal antibody or its fragment mentioned above.

If this reagent system is combined with the reagent system of the present invention, one test kit is provided which is very convenient for medical diagnosis for the immunological determination of both human $\alpha_2$-PI and human plasmin/$\alpha_2$-PI complex in an assay sample.

The following examples illustrate the present invention.

EXAMPLE 1

(1) Preparation of human $\alpha_2$-PI

In accordance with the method of Aoki and Moroi cited hereinabove, 7.7 mg of human $\alpha_2$-PI was obtained from 2,360 ml of human plasma

(2) Immunization of mice

Male Balb/c mice were immunized intraperitoneally with an emulsion of 100 micrograms of human $\alpha_2$-plasmin inhibitor and complete Freund's adjuvant twice at an interval of 21 days. Seven days and 88 days later, 30 micrograms of human $\alpha_2$-PI in physiological saline was additionally administered intravenously. Four days after the final immunization, the spleen cells were isolated for cell fusion.

(3) Preparation of a suspension of the spleen cells

The spleen cells were taken out aseptically and passed through a stainless steel mesh to obtain a suspension of the spleen cells. The cells were transferred to RPMI-1640 medium (a product of GIBCO) supplemented with 0.39 g/liter of L-glutamine, 0.2 g/liter of kanamycin sulfate and 2.0 g/liter of $NaHCO_3$. The cells which proliferated were washed three times with RPMI-1640 and again suspended in RPMI-1640 medium.

(4) Preparation of myeloma cells

Mouse myeloma cells, P3-Ul, were cultivated in RPMI-1640 medium supplemented with 0.39 g/liter of L-glutamine, 0.2 g/liter of kanamycin sulfate, 2.0 g/liter of $NaHCO_3$ and 10% fetal calf serum (to be abbreviated as 10% FCS-RPMI-1640). At the time of cell fusion, the myeloma cells were in the log phase of cell fission.

(5) Cell fusion

The spleen cells and the myeloma cells were suspended in a ratio of 10:1 in a serum-free RPMI-1640 medium, and then centrifuged at about 200 G for 5 minutes. The supernatant was removed, and the sediment was incubated together with 1 ml of [a 50% solution of polyethylene glycol having an average molecular weight of 1,540 (pH 8.2)] at 37°C for 2 minutes. Then, 9 ml of serum-free RPMI-1640 medium was added, and the cells were again suspended carefully for 5 minutes. The suspension was centrifuged at about 200G for 5 minutes, and again suspended in 10% FCS-RPMI-1640 medium so that a concentration of $8 \times 10^6$ cells/ml was obtained. The suspension was then distributed on a 96-microwell plate (about 100 microliters per well). The fused cells were cultivated at 37°C using 5% $CO_2$.

(6) Selection and cultivation of fused cells capable of producing an antibody to human $\alpha_2$-PI

One day after cell fusion, HAT medium was added in an amount of 100 microliters per well. Thereafter, at intervals of 2 days, one half of the medium was exchanged with a fresh supply of HAT medium, and the cultivation was continued. Eight days later, the supernatant of the culture fluid of hybridoma cells was screened for antibodies to human $\alpha_2$-PI by the enzyme linked immunosorbent assay. The antigen used in the screening was human $\alpha_2$-PI, and the second antibody was alkali phosphatase-conjugated rabbit anti-mouse antibodies.

349 wells in total were found to be positive by the enzyme-linked immunosorbent assay, and thus to produce antibodies specific to $\alpha_2$-PI.

When it was observed that the proliferation of the cells was active, HT medium was added. The medium was exchanged with HT medium four times at intervals of one day. Thereafter, the cultivation was carried out by using ordinary 10% FCS-RPMI-1640 medium.

EXAMPLE 2

Selection of fused cells producing antibodies to human $\alpha_2$-PI:-

The fused cells producing antibodies to human $\alpha_2$-PI was screened by the following procedure for fused cells which had the action of suppressing the fibrinolysis inhibiting activity of human $\alpha_2$-PI.

The fused cells in each well were cultivated in 10% FCS-RPMI-1640 medium until the number of the cells reached about $2 \times 10^7$. The cells were then centrifuged at about 200 G for 5 minutes. The supernatant was removed, and the cells were washd with 10 ml of a serum-free RPMI-1640 medium, and further centrifuged at about 200G for 5 minutes. The supernatant was removed, and the cells were suspended in 10 ml of a mixed serum-free medium (to be abbreviated as "MITES medium") composed of RPMI-1640 medium supplemented with 5.0 ml/liter of 2-mercaptoethanol, 7.5 ml/liter of insulin, 5.0 ml/liter of transferrin, 5.0 ml/liter of ethanolamine, 5.0 ml/liter of sodium selenite, 0.39 g/liter of L-glutamine, 0.2 g/liter of kanamycin sulfate, and 2.38 g/liter of Hepes, Dulbecco's MEM, and Ham's F-12 (2:1:1), and cultivated for 3 days.

The supernatant of the culture fluid was recovered, and concentrated to 25 times. To 25 microliters of the concentrate was added 0.4 microgram of human $\alpha_2$-PI, and the mixture was incubated at 37ºC for 30 minutes. Then, 0.025 unit of plasminogen and 0.031 unit of urokinase were added, and the amount of the entire solution was adjusted to 40 microliters. Ten microliters of it was placed on a fibrin plate. The fibrin plate was left to stand at a temperature of 37ºC and a humidity of more than 95% for 18 hours, and the area of fibrin dissolved was measured.

The results show that the fibrinolysis inhibiting activity of human $\alpha_2$-PI added to the antibodies produced by ID10 fused cells was completely suppressed.

EXAMPLE 3

Cloning of fused cells:-

The fused cells (IDl0) which were found to be positive in the test for the activity of the antibodies to human $\alpha_2$-PI were cloned by the following procedure.

The ID10 cells were diluted so that each well of a 96-well microtiter plate contained 0.9 cell. Thymus cells of Balb/c mice were added as feeder cells and distributed on the plate and cultivated in 10% FCS-RPMI-1640 medium. Microscopic observation exactly showed single cell colonies formed. The supernatant of the culture fluid of the fused cells was screened by the enzyme-linked immunosorbent assay for antibodies to human $\alpha_2$-PI.

Twenty-six wells in total were found to be positive by the enzyme-linked immunosorbent assay, and thus to produce monoclonal antibodies to human $\alpha_2$-PI.

Purification of monoclonal antibodies:-

In order to produce large amounts of monoclonal antibodies to human $\alpha_2$-PI, about $10^7$ fused cells were intraperitoneally injected into Balb/c mice pre-treated with pristane. About one week later, the antibodies were isolated from the ascites fluid and purified by the method of Ey et al. [P. L. Ey, S. J. Prowse and C. R. Jenkin, Immunochemistry, 15, 429-436 (1978)]. Twenty milligrams of monoclonal antibodies to human $\alpha_2$-PI were obtained from 2.5 ml of the ascites fluid.

Characterization of the purified monoclonal antibodies:-

The particular classes of the purified monoclonal antibodies were determined by the Ouchterlony gel diffusion test using class-specific antimouse-immunoglobulin antisera. The results given in Table 1 show that many of the antibodies to human $\alpha_2$-PI are of the H-chain $\gamma_1$ type and L-chain $x$ type.

Table 1

| Antibody | IgG$_1$ | IgG$_2$a | IgG$_2$b | IgM | $x$ |
|---|---|---|---|---|---|
| IBI0C4 | | + | | | + |
| IBI0GII | | + | | | + |
| IDI0CI | + | | | | + |
| IDI0FI0 | + | | | | + |
| IDI0-IF5 | + | | | | + |
| IDI0BII | + | | | | + |
| IDI0-2H8 | + | | | | + |
| 2HI2D5 | | | + | | + |

## EXAMPLE 4

Purification of human plasmin/$\alpha_2$-PI complex:-

One liter of a human plasma sample was filtered on a filter paper, and passed through a column (300 ml) of Lys-Sepharose to obtain 1050 ml of plasminogen-depleted plasma. Plasminogen (9.3 mg) was freshly added to a portion (250 ml) of this plasma sample, and the mixture was stirred at room temperature. Urokinase (228,000 units in total), in seven divided portions, was added over the course of 15 hours at 4°C. The mixture was further stirred at room temperature for 4 hours, and 1 mM DFP (di-isopropyl fluorophosphate), 10 units/ml aprotinin and 5 mM benzamidine (the concentrations being the final concentrations) were added, and the mixture was stirred at room temperature for 1 hour. The mixture was charged onto a column of Lys-Sepharose having a gel volume of 50 ml. The column was washed with 150 ml of Tris-buffered saline containing 10 units/ml aprotinin, 400 ml of 1.0-M NaCl-Tris buffer containing 10 units/ml aprotinin, and further with 100 ml of Tris-buffered saline containing 10 units/ml aprotinin. By a linear gradient elution method in which the concentration of epsilon-aminocaproic acid was raised from 0 to 25 mM, the human plasmin/$\alpha_2$-PI complex was eluted from the Lys-Sepharose column. The resulting crude human plasmin/$\alpha_2$-PI complex sample was subjected to HPLC on a hydroxyapatite column, and by a linear gradient from 75 mM phosphate buffer (pH 6.8) to 0.1M NaCl and 200 mM phosphate buffer, 3.65 mg of pure human plasmin/$\alpha_2$-PI complex was obtained.

## EXAMPLE 5

Determination of antigen epitopes which the monoclonal antibody can reccognize and combine with:-

A solution of $\alpha_2$-PI and a solution of plasmin/$\alpha_2$-PI complex prepared so that the final concentration of SDS was 1% were each heated at 100°C for 3 minutes to prepare samples for SDS gel electrophoresis. Each of the samples was applied in an amount of 0.5 microgram/lane to a 7.5%-20% gradient gel prepared in advance, and subjected to electrophoresis at room temperature for about 2.5 hours. Immediately then, the gel was laid over a nitrocellulose membrane filter, and in a solution composed of 25 mM Tris, 192 mM glycine, 20% methanol and 0.01% SDS having a pH of 8.3, western blotting was carried out at a fixed voltage of 50 V for 2 hours at 4°C to fix the proteins separated by the SDS-gel to the nitrocellulose membrane filter.

The nitrocellulose membrane filter was put in a solution containing 50 mM Tris-HCl and 0.15 M NaCl and 3% gelatin at a pH of 7.4, and blocking was carried out. The nirrocellulose membrane filter was cut along the lanes, and the cut frgments were put in three types of anti-$\alpha_2$-PI monoclonal antibody (IDI0CI, 2HI2D5 and IBI0GII) solutions (antibody concentration 2 micrograms/ml) and reacted over-night at room temperature. The nitrocellulose membrane filter fragments were washed three times with a solution of 50mM Tris-HCl and 0.15M NaCl containing 0.05% Tween 20 at a pH of 7.4 and reacted at room temperature for 2 hours with a goat anti-mouse IgG solution labelled with peroxidase. After washing with the aforesaid solution containing Tween 20, a substrate was added, and 2 or 3 minutes later, the reaction product was washed and the reaction was stopped.

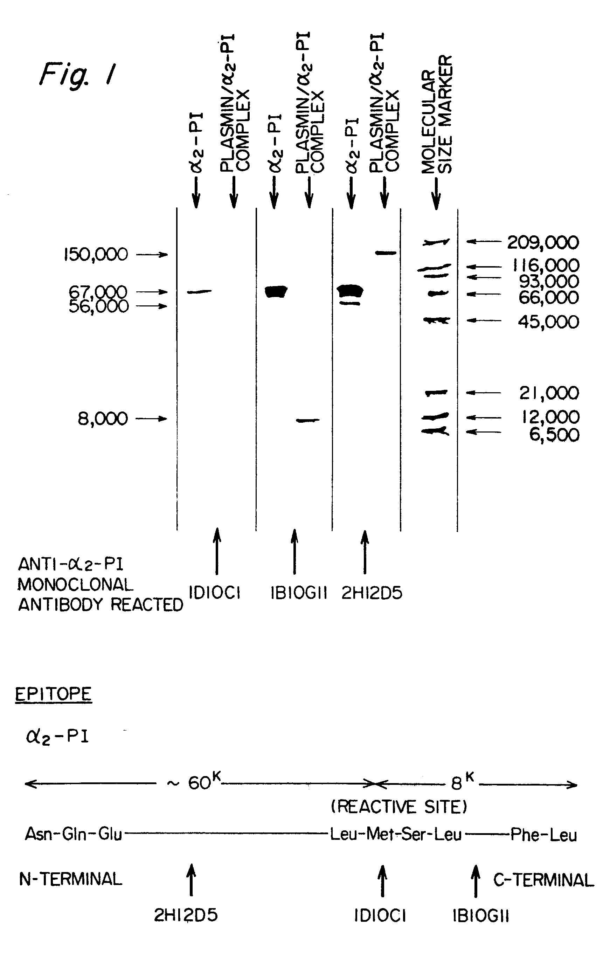

The results are shown in Figure 1.

The experimental results suggest with which epitopes of the $\alpha_2$-PI and plasmin/$\alpha_2$-PI complex protein molecules the three types of monoclonal anitbodies will combine.

When plasmin forms a complex with $\alpha_2$-PI, a peptide having a molecular weight of about 8000 on the C-terminal side of the $\alpha_2$-PI is cut, but this peptide combines with the complex with a certain kind of force and is liberated from the complex by sodium dodecylsulfate (SDS) [see, for example, B. Wiman and D. Collen: The Journal of Biological Chemistry, 254, 9291-9279)].

It is known that $\alpha_2$-PI having a molecular weight of 67,000 is cleaved with leukocyte elastase to be converted to two forms having a molecular weight of 56,000 and 11,000 (see M. S. Brower and P. C. Harpel: The Journal of Biological Chemistry, 257, 9849-9854, 1982).

The anti-$\alpha_2$-PI monoclonal antibody IDI0CI reacted and combined with $\alpha_2$-PI having a molecular weight of 67000, but its combination with the complex was not observed. The present inventors already found by the fibrin plate method and an assay using synthetic substrate S-2251 that IDI0CI antibody recognizes, and combines with, the reactive site of $\alpha_2$-PI. As a result of blotting, no combination with the complex was observed. This is presumably because SDS resulted in the separation of the peptide having a molecular weight of 8000 on the C-terminal side of $\alpha_2$-PI.

On the other hand, the IBI0GII antibody combined with $\alpha_2$-PI having a molecular weight of 67000, and interestingly, reacted and combined with the peptide having a molecular weight of 8000 which was liberated from the complex by SDS.

The 2HI2D5 antibody combined with $\alpha_2$-PI having a molecular weight of 67,000 and $\alpha_2$-PI having a molecular weight of 56,000 treated with leukocyte elastase, and also with the complex.

EXAMPLE 6

Measurement of the dissociation constant of a monoclonal antibody:-

Antigen $\alpha_2$-PI or plasmin/$\alpha_2$-PI complex, in a concentration of 10 micrograms/ml, was added to a 96-well plate at a rate of 1000 microliters per well, and fixed at 4°C overnight.

Blocking was carried out using phosphate buffered saline (PBS) containing 1% BSA, and then a $^{125}$I-labelled monoclonal antibody diluted to various concentrations (for example 1 - 10 nM) was added in an amount of 100 microliters per well, and reacted with the fixed antigen at 37°C for 4 hours. Finally, the plate was washed with PBS containing 0.05% Tween 20. Then, each of the well was cut out and put in a tube, and its $^{125}$I radioactivity was measured by a gamma-counter.

The $^{125}$I radioactivity of 100 microliter of the $^{125}$I-labelled monoclonal antibody in each of the concentrations was measured, and made the total count (cpm). The count of the cut-out well was defined as the count of the antibody bound to the fixed antigen. The difference between the total count of the $^{125}$I-labelled antibody and the count of the bound $^{125}$I-labelled antibody was defined as the count of the free $^{125}$I-labelled antibody.

The ratio of the $^{125}$I-labelled antibody bound to the antigen and the free $^{125}$I-labelled antibody was plotted on the axis of ordinate, and the concentration (nM) of the $^{125}$I-labelled antibody bound to the antigen, on the axis of abscissa. The reciprocal of two times the slope of the curve obtained was defined as Kd (dissociation constant.

See, for example, M. E. Frankel and W. Gerhard: Molecular Immunology, 16, 101-106 (1979).

The Kd values of the three anti-$\alpha_2$-PI monoclonal antibodies (IDI0CI, IBI0GII, and 2HI2D5) against the antigen $\alpha_2$-PI and plasmin/$\alpha_2$-PI complex are summarized in Table 2.

## Table 2

| Antigen / Monoclonal antibody | $\alpha_2$-PI | Plasmin/$\alpha_2$-PI complex |
|---|---|---|
| ID10C1 | $2.18 \times 10^{-9}$ | $1.06 \times 10^{-8}$ |
| IB10G11 | $2.89 \times 10^{-9}$ | $3.17 \times 10^{-9}$ |
| 2H12D5 | $1.54 \times 10^{-9}$ | $2.59 \times 10^{-9}$ |

EXAMPLE 7

Method of measuring plasmin/$\alpha_2$-PI complex:-

A rabbit anti-human plasminogen antibody (polyclonal) diluted to a concentration of 20 micrograms/ml was added to a 96-well microtiter plate at a rate of 100 microliters per well, and fixed overnight at 4°C.

A phosphate-buffered saline (PBS) solution containing 1% BSA was added in an amount of 150 microliters per well, and left to stand at 37°C for 2 hours to carry out blocking. The plate was washed three times with PBS containing 0.05% Tween 20, and $\alpha_2$-PI and plasmin/$\alpha_2$-PI complex, diluted to various concentrations, were each added in an amount of 100 microliters per well, and reacted at 37°C for 2 hours with the antibodies (primary antibodies) fixed to the wells. After washing, an alkaline phosphatase-labelled anti-$\alpha_2$-PI monoclonal antibody IB10G11 was added in a concentration of 350 micrograms/ml at a rate of 100 microliters per well, and incubated at 37°C for 2 hours. The plate was washded, and a substrate in a concentration of 1 mg/ml was added in an amount of 100 microliters per well, and changes in absorbance ($\Delta$A 405 nm/min.) at a wavelength of 405 nm were measured by a microplate photometer.

Incidentally, before using the anti-plasminogen antibody sample in this measuremnt, it was charged on $\alpha_2$-PI-Sepharose to absorb anti-$\alpha_2$PI antibody contained in a trace amount in the sample.

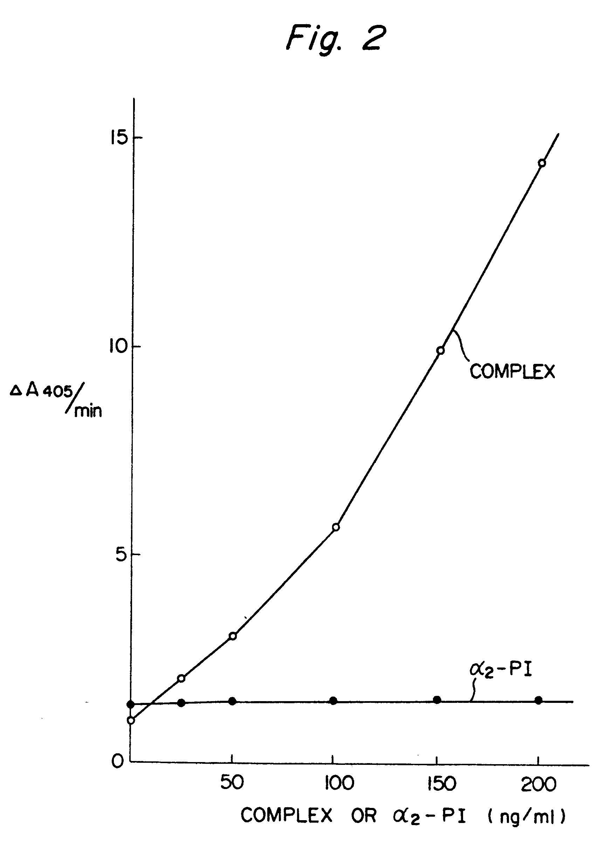

Figure 2 shows the relation between the concentration (ng/ml) of the plasmin/$\alpha_2$-PI complex or $\alpha_2$-PI and the change in absorbance ($\Delta$A 405 nm/min.).

The relation between the concentration of plasmin/$a_2$-PI complex and the change in absorbance is nearly linear, and by using this graph as a calibration curve, the plasmin/$\alpha_2$-PI complex in an assay sample can be mesured. This assay system does not detect free $\alpha_2$-PI.

## Claims

1. A method for immunologically determining a human plasmin/$\alpha_2$-plasmin inhibitor complex in an assay sample by using a primary antibody fixed to an insoluble solid carrier and a labelled secondary antibody, wherein the secondary antibody is a monoclonal antibody or its fragment specific to both a human $\alpha_2$-plasmin inhibitor and a human plasmin/$\alpha_2$-plasmin inhibitor complex, and the primary antibody is a polyclonal antibody specific to human plasmin.

2. The method of claim 1 wherein the labelled secondary antibody is labelled with an enzyme, a luminescent substance or a radioisotope.

3. The method of claim 1 wherein the labelled secondary antibody and the assay sample are contacted simultaneously with the primary antibody fixed to the insoluble solid carrier.

4. The method of claim 1 wherein the monoclonal antibody is a monoclonal antibody specific to a fragment of a human $\alpha_2$-plasmin inhibitor molecule which has a length extending about 12,000 in molecular weight from the carboxylic terminal of said inhibitor molecule.

5. A reagent system for immunological determination of a human plasmin/$\alpha_2$-plasmin inhibitor complex in an assay sample comprising a primary antibody fixed to an insoluble solid carrier and a labelled secondary antibody, wherein the secondary antibody is a monoclonal antibody or its fragment specific to both $\alpha_2$-plasmin inhibitor and a human plasmin/$\alpha_2$-plasmin inhibitor complex, and the primary antibody is a polyclonal antibody specific to human plasmin.

6. The reagent system of claim 5 wherein the insoluble solid carrier is a plastic receptacle, plastic beads, glass beads or metal particles.

7. The reagent system of claim 5 wherein the labelled antibody is labelled with an enzyme, a luminescent substance or a radioisotope.

8. The reagent system of claim 5 wherein the monoclonal antibody is a monoclonal antibody specific to a fragment of a human $\alpha_2$-plasmin inhibitor molecule which has a length extending about 12,000 in molecular weight from the carboxyl terminal of said inhibitor molecule.

**Patentansprüche**

1. Verfahren zur immunologischen Bestimmung eines menschlichen Plasmin/$\alpha_2$-Plasmininhibitorkomplexes in einer Testprobe unter Verwendung eines an einen unlöslichen festen Träger fixierten ersten Antikörpers und eines markierten zweiten Antikörpers, dadurch **gekennzeichnet,** daß der zweite Antikörper ein monoklonaler Antikörper oder ein Fragment davon, der bzw. das spezifisch sowohl für einen menschlichen $\alpha_2$-Plasmininhibitor als auch einen menschlichen Plasmin/$\alpha_2$-Plasmininhibitorkomplex ist, und der erste Antikörper ein polyklonaler Antikörper, der für menschliches Plasmin spezifisch ist, ist.

2. Verfahren nach Anspruch 1, dadurch **gekennzeichnet,** daß der markierte zweite Antikörper mit einem Enzym einer lumineszenten Substanz oder einem Radioisotop markiert ist.

3. Verfahren nach Anspruch 1, dadurch **gekennzeichnet,** daß der markierte zweite Antikörper und die Testprobe gleichzeitig mit dem auf einen unlöslichen festen Träger fixierten ersten Antikörper in Kontakt gebracht werden.

4. Verfahren nach Anspruch 1, dadurch **gekennzeichnet,** daß der monoklonale Antikörper ein monoklonaler Antikörper, der spezifisch für ein Fragment des menschlichen $\alpha_2$-Plasmininhibitormoleküls ist, das eine Länge, die sich etwa um 12.000 bezogen auf das Molekulargewicht von dem carboxylterminalen Ende des Inhibitormoleküls erstreckt, besitzt, ist.

5. Reagenzsystem zur immunologischen Bestimmung eines menschlichen Plasmin/$\alpha_2$-Plasmininhibitorkomplexes in einer Testprobe, umfassend einen an einen unlöslichen festen Träger fixierten ersten Antikörper und einen markierten zweiten Antikörper, dadurch **gekennzeichnet,** daß der zweite Antikörper ein monoklonaler Antikörper oder dessen Fragment, der bzw. das spezifisch sowohl für den $\alpha_2$-Plasmininhibitor als auch den menschlichen Plasmin/$\alpha_2$-Plasmininhibitorkomplex ist, und der erste Antikörper ein monoklonaler Antikörper, der spezifisch für menschliches Plasmin ist, ist.

6. Reagenzsystem nach Anspruch 5, dadurch **gekennzeichnet,** daß der unlösliche, feste Träger ein Plastikbehälter, Plastikkügelchen, Glaskügelchen oder Metallteilchen ist.

7. Reagenzsystem nach Anspruch 5, dadurch **gekennzeichnet,** daß der markierte Antikörper mit einem Enzym, einer lumineszenten Substanz oder einem Radioisotop markiert ist.

8. Reagenzsystem nach Anspruch 5, dadurch **gekennzeichnet,** daß der monoklonale Antikörper ein monoklonaler Antikörper ist, der spezifisch für ein Fragment eines menschlichen $\alpha_2$-Plasmininhibitormoleküls ist, das eine Länge, die sich um etwa 12.000 bezogen auf das Molekulargewicht von dem carboxylterminalen Ende des Inhibitormoleküls erstreckt, besitzt, ist.

EP 0 169 549 B1

**Revendications**

1. Méthode de dosage immunologique d'un complexe plasmine/inhibiteur de plasmine $\alpha_2$ humain dans un échantillon à analyser, par utilisation d'un anticorps primaire fixé à un support solide insoluble et d'un anticorps secondaire marqué, dans laquelle
l'anticorps secondaire est un anticorps monoclonal ou son fragment spécifique envers chacun d'un inhibiteur de plasmine $\alpha_2$ humain et d'un complexe plasmine/inhibiteur de plasmine $\alpha_2$ humain, et l'anticorps primaire est un anticorps polyclonal spécifique envers la plasmine humaine.

2. Méthode selon la revendication 1, dans laquelle l'anticorps secondaire marqué est marqué par une enzyme, une substance luminescente ou un radio-isotope.

3. Méthode selon la revendication 1, dans laquelle l'anticorps secondaire marqué et l'échantillon à analyser sont mis en contact simultanément avec l'anticorps primaire fixé au support solide insoluble.

4. Méthode selon la revendication 1, dans laquelle l'anticorps monoclonal est un anticorps monoclonal spécifique envers un fragment d une molécule d'inhibiteur de plasmine $\alpha_2$ humain qui s'étend sur une longueur correspondant à un poids moléculaire d'environ 12 000 à partir de l'extrémité carboxylique de ladite molécule d'inhibiteur.

5. Un système de réactifs pour le dosage immunologique d'un complexe plasmine/inhibiteur de plasmine $\alpha_2$ humain dans un échantillon à analyser, comprenant un anticorps primaire fixé à un support solide insoluble et un anticorps secondaire marqué, dans lequel
l'anticorps secondaire est un anticorps monoclonal ou son fragment spécifique envers chacun d'un inhibiteur de plasmine $\alpha_2$ et d'un complexe plasmine/inhibiteur de plasmine $\alpha_2$ humain, et l'anticorps primaire est un anticorps polyclonal spécifique envers la plasmine humaine.

6. Système de réactifs selon la revendication 5, dans lequel le support solide insoluble est un récipient en matière plastique, des perles de matière plastique, des perles de verre ou des particules métalliques.

7. Système de réactifs selon la revendication 5, dans lequel l'anticorps marqué est marqué par une enzyme, une substance luminescente ou un radio-isotope.

8. Système de réactifs selon la revendication 5, dans lequel l'anticorps monoclonal est un anticorps monoclonal spécifique envers un fragment d'une molécule d'inhibiteur de plasmine $\alpha_2$ humain qui s'étend sur une longueur correspondant à un poids moléculaire d'environ 12 000 à partir de l'extrémité carboxylique de ladite molécule d'inhibiteur.

13

Fig. 1

EPITOPE

## Fig. 2